# EUROPÄISCHE PATENTSCHRIFT

(11) **EP 4 507 751 B1**
(45) Veröffentlichungstag und Bekanntmachung des Hinweises auf die Patenterteilung: **28.01.2026**
(21) Anmeldenummer: 23721831.8
(22) Anmeldetag: 13.04.2023
(51) Int. Cl.: A61L 31/02, A61L 31/08, A61L 31/14, A61L 31/18

(54) **RÖNTGENDICHTE BESCHICHTUNG FÜR ENDOVASKULÄRE VORRICHTUNGEN**
RADIOPAQUE COATING FOR ENDOVASCULAR DEVICES
REVÊTEMENT RADIO-OPAQUE POUR DISPOSITIFS ENDOVASCULAIRES

(30) Priorität: 13.04.2022 DE 102022109090
(43) Veröffentlichungstag der Anmeldung: 19.02.2025
(73) Patentinhaber: Phenox GmbH, 44801 Bochum (DE)
(72) Erfinder: SCHLAGKAMP, Simon, 46282 Dorsten (DE); ASCHERFELD, Jörg, 45529 Hattingen (DE); TRÖSKEN, Volker, 58456 Witten (DE)
(74) Vertreter: Schneiders & Behrendt Bochum
(86) Internationale Anmeldenummer: PCT/EP2023/059620
(87) Internationale Veröffentlichungsnummer: WO 2023/198808

(56) Entgegenhaltungen:
- EP-A2- 1 265 651
- DE-A1- 102007 030 751
- US-A1- 2005 096 722

## Beschreibung

Die Erfindung betrifft eine röntgendichte Beschichtung für endovaskuläre Vorrichtungen, wobei die Beschichtung selektiv auf die Vorrichtung aufgebracht wird, um die mechanischen Eigenschaften der Vorrichtung durch die Beschichtung so wenig wie möglich zu beeinflussen.

Endovaskuläre Vorrichtungen wie beispielsweise Stents und andere Implantate, Filter, Thrombektomievorrichtungen oder Flow Diverter sind in der Regel sehr filigrane Objekte. Für eine erfolgreiche Platzierung und Anwendung im Gefäßsystem ist es wichtig, ihre genaue Position bereits während der Intervention im Röntgenbild zu erkennen. Aufgrund ihrer geringen Masse und der verwendeten Materialien zeigen viele endovaskuläre Vorrichtungen jedoch im Röntgenbild an sich einen nur geringen Kontrast.

Entsprechend umfassen endovaskuläre Vorrichtungen häufig Marker aus röntgendichten Materialien wie beispielsweise Gold oder Platin, die dem behandelnden Arzt eine bessere Visualisierung der Vorrichtung während der Intervention unter Röntgendurchleuchtung ermöglichen. Entsprechende Marker markieren jedoch meistens nur bestimmte Bereiche der Vorrichtung, wie beispielsweise den Anfang oder das Ende eines Stents.

EP 1 265 651 A2 betrifft die selektive Beschichtung endovaskulärer Stent mit einem röntgendichten Material, wobei die selektive Beschichtung insbesondere Stents für Bifurkationen betrifft und eine Identifikation der Fenestrierung während der Intervention ermöglichen soll. Entsprechend schlägt das Dokument vor allem eine Beschichtung der Enden und der Fenestrierung des Stents vor.

Grundsätzlich wäre es aber wünschenswert, dass nicht nur bestimmte Bereiche der Vorrichtung besser im Röntgenbild visualisiert werden, sondern möglichst die gesamte Vorrichtung sichtbar ist. So gibt die alleinige Visualisierung der Endbereiche einer Vorrichtung wenig oder keinen Aufschluss darüber, ob diese beispielsweise auch im dazwischenliegenden Bereich anforderungsgemäß expandiert ist.

Um eine verbesserte Röntgensichtbarkeit der Vorrichtung zu erzielen, kann auch die gesamte Vorrichtung mit einem entsprechenden röntgendichten Material beschichtet werden. Dabei verändern die Vorrichtungen je nach verwendetem Werkstoff und verwendetem röntgendichten Material jedoch auch ihre mechanischen Eigenschaften.

Konventionelle Werkstoffe wie etwa Stahl oder Gold zeigen beispielsweise ein lineares Elastizitätsverhalten, das heißt Spannungen und Dehnungen des Materials nehmen bei Belastung bis zum Erreichen der Elastizitätsgrenze proportional zu. In diesem Bereich nimmt der belastete Werkstoff nach dem Entfernen der Belastung seine ursprüngliche Form auch wieder ein. Erhöht man die Belastung und führt die Spannung beziehungsweise Dehnung über die Elastizitätsgrenze hinaus, kommt es zu einer irreversiblen plastischen Verformung.

Pseudoelastische Legierungen, auch Formgedächtnislegierungen genannt, zeichnen sich dagegen durch ein nicht-lineares Elastizitätsverhalten aus. Sie besitzen die Eigenschaft, selbst nach beträchtlicher Verformung bei Entlastung in ihre Ausgangsform zurückzukehren.

Solche pseudoelastischen Legierungen wie beispielsweise Nitinol, NiTiCu, CuZn, CuZnAl oder CuAINi können dabei bis zu zehnmal stärker gedehnt werden als herkömmliche Federstähle, ohne bleibend verformt zu werden.

Dabei durchläuft das Material eine Spannungs-Dehnungs-Hysterese mit einem Belastungs- und einem Entlastungsplateau.

Bei endovaskulären Vorrichtungen, deren Grundgerüst aus pseudoelastischen Legierungen besteht, hat eine komplette Beschichtung, die auch ein röntgendichtes Material umfassen kann, zumindest einen entscheidenden Nachteil. Die aufgebrachte Schicht schränkt die Pseudoelastizität des Werkstoffs mit zunehmender Schichtdicke immer stärker ein. Wichtige Faktoren wie beispielsweise Radialkräfte und Aufstellkräfte der Vorrichtung oder Vorschubkräfte im Mikrokatheter sind hiervon betroffen.

Entsprechend muss insbesondere bei der Beschichtung von endovaskulären Vorrichtungen aus Formgedächtnislegierungen immer ein Kompromiss aus guter Sichtbarkeit - durch eine höhere Schichtdicke - und mechanischer Performance - durch eine niedrigere Schichtdicke - gefunden werden.

Es ist daher Aufgabe der Erfindung, eine Beschichtung sowie ein Beschichtungsverfahren für endovaskuläre Vorrichtungen aus Formgedächtnislegierungen zur Verfügung zu stellen, die die Nachteile bekannter Beschichtungen nicht aufweisen und die eine verbesserte Röntgensichtbarkeit der endovaskulären Vorrichtungen ermöglichen, ohne dass hierdurch die mechanischen Eigenschaften der endovaskulären Vorrichtungen unangemessen beeinträchtigt werden.

Gelöst wird diese Aufgabe durch die Erfindung mit den Merkmalen der Ansprüche 1 und 16 sowie ein Verfahren mit den Merkmalen der Ansprüche 6 und 9. Vorteilhafte Ausgestaltungen sind jeweils Gegenstand der abhängigen Ansprüche. Es ist darauf hinzuweisen, dass die in den Ansprüchen einzeln aufgeführten Merkmale auch in beliebiger und technologisch sinnvoller Weise miteinander kombiniert werden können und somit weitere Ausgestaltungen der Erfindung aufzeigen.

Wesentliches Ziel der Erfindung ist es, einen Kompromiss zwischen einer möglichst vollständigen röntgendichten Beschichtung zu finden, die eine weitestgehend vollständige Sichtbarkeit der Vorrichtung im Röntgenbild ermöglicht und die gleichzeitig die mechanischen Eigenschaften der Vorrichtung möglichst wenig beeinflusst.

Der wesentliche Erfindungsgedanke beruht somit darauf, nur die Bereiche der Vorrichtung zu beschichten, die für die mechanischen Eigenschaften der Vorrichtung weitestgehend zu vernachlässigen sind. Versuche haben gezeigt, dass dies jene Bereiche der Vorrichtung sind, die bei der Verwendung der Vorrichtung nur eine geringe Dehnung erfahren.

Zu den in diesem Zusammenhang wichtigen mechanischen Eigenschaften einer endovaskulären Vorrichtung gehören dabei einerseits die äußeren Einflüssen entgegenwirkende *radial resistive force* (RRF), also die Kraft, die die Vorrichtung den von außen einwirkenden Kräften entgegensetzt, und die von der Vorrichtung selbst ausgehende *chronic outward force* (COF), die die Vorrichtung während und nach der Freisetzung auf das umliegende Gewebe, beispielsweise die Gefäßwand, ausübt.

Für eine Vorrichtung aus einer Formgedächtnislegierung ergeben sich aus den beiden Größen RRF und COF verschiedene Überlegungen, denn der Durchmesser des Zielgefäßes, in dem die Vorrichtung eingesetzt wird, ist in der Regel kleiner als der Durchmesser der Vorrichtung in ihrem frei entfalteten Zustand.

Wird die zunächst komprimierte Vorrichtung aus ihrem Transportsystem, beispielsweise dem Zuführkatheter, freigesetzt, expandiert diese solange, bis sie in Kontakt mit der Gefäßwand tritt, die ihre weitere Expansion verhindert. Da die Vorrichtung das Bestreben hat, in ihre Ausgangsform zurück zu gelangen, wirkt an dieser Stelle eine geringe kontinuierliche Kraft COF auf die Gefäßwand. Je geringer dabei der Durchmesser der Vorrichtung ist, also je geringer die radiale Expansion der Vorrichtung nach der Freisetzung ist, desto größer ist die COF, die auf das Gefäß wirkt.

Eine andere wichtige zu nennende Größe ist die RRF. RRF beschreibt die Kraft, die benötigt wird, um beispielsweise durch Drücke von außen die Vorrichtung wieder auf einen geringeren Durchmesser zu komprimieren.

Bei der Lagerung oder bei der Anwendung der Vorrichtung werden nun einzelne Bereiche über die Elastizitätsgrenze eines konventionellen Beschichtungsmaterials wie beispielsweise Gold, das als röntgendichter Marker auf der Vorrichtung aufgebracht sein kann, gedehnt. Im Gegensatz zu der Formgedächtnislegierung der Vorrichtung wird diese röntgendichte Schicht aus konventionellem Beschichtungsmaterial somit dauerhaft plastisch verformt. Diese Verformung behindert entsprechend mit zunehmender Schichtdicke die Pseudoelasitizität des Grundgerüstes der Vorrichtung und folglich wichtige Leistungsmerkmale der Vorrichtung wie RRF und COF.

So kann zum Beispiel die gewünschte COF nicht mehr erreicht werden oder es muss eine hohe RRF überwunden werden, um die Vorrichtung (wieder) in das Transportsystem oder den Mikrokatheter einzuführen.

Bei der erfindungsgemäßen selektiven Beschichtung werden entsprechend nur die Bereiche oder Abschnitte der Vorrichtung beschichtet, die nicht oder nur vernachlässigbar wenig zur mechanischen Performance, wie zum Beispiel COF und RRF, beitragen. Dies sind Bereiche, welche bei Lagerung, Einbringung in den Patienten und bei der Anwendung wenig mechanische Spannungen und Verformungen aufweisen. Auf diese Weise kann die Röntgensichtbarkeit durch hohe Schichtdicken auf solchen Bereichen der Vorrichtung verbessert werden, die die mechanische Performance nicht oder nur vernachlässigbar wenig beeinflussen. Es muss also nicht auf die Vorteile der Formgedächtnislegierung verzichtet werden.

Die Erfindung betrifft entsprechend eine endovaskuläre Vorrichtung aus pseudoelastischen Legierungen, umfassend eine selektive Beschichtung aus röntgendichten Materialien, wobei die Beschichtung nur Bereiche oder Abschnitte der endovaskulären Vorrichtung umfasst, die im radial maximal belasteten Zustand der Vorrichtung eine Dehnung in dem entsprechenden Bereich von unter 5 %, vorzugsweise von unter 3 %, noch bevorzugter von unter 1,5 % und insbesondere von unter 1 % gegenüber der Vorrichtung im radial unbelasteten Zustand aufweisen.

Anders formuliert umfasst die selektive Beschichtung Bereiche und Abschnitte der endovaskulären Vorrichtung nicht, die im radial maximal belasteten Zustand der Vorrichtung eine Dehnung von mindestens 5 %, vorzugsweise von mindestens 3 %, noch bevorzugter von mindestens 1,5 % und insbesondere von mindestens 1 % gegenüber der Vorrichtung im radial unbelasteten Zustand aufweisen.

Dabei hat sich gezeigt, dass durch die Beschränkung der Beschichtung auf die Bereiche oder Abschnitte, die im radial maximal belasteten Zustand der Vorrichtung eine Dehnung von unter 5 %, vorzugsweise von unter 3 %, noch bevorzugter von unter 1,5 % und insbesondere von unter 1 % gemäß Definition aufweisen, eine ausreichende Verbesserung der Röntgensichtbarkeit der Vorrichtung bei gleichzeitiger Erhaltung der wichtigen mechanischen Eigenschaften erzielbar ist.

Vorzugsweise umfasst die Beschichtung ferner nur Bereiche oder Abschnitte der endovaskulären Vorrichtung, die sowohl im radial maximal belasteten Zustand der Vorrichtung als auch im radial nominal belasteten Zustand eine Dehnung in dem entsprechenden Bereich von unter 5 %, vorzugsweise von unter 3 %, noch bevorzugter von unter 1,5 % und insbesondere von unter 1 % gegenüber der Vorrichtung im radial unbelasteten Zustand aufweisen.

Unter der Dehnung der Vorrichtung ist definitionsgemäß die relative Änderung der Länge eines Bereichs der Vorrichtung in einem radial belasteten Zustand, beispielsweise dem radial maximal oder radial nominal belasteten Zustand, bezogen auf den radial unbelasteten Zustand zu verstehen. Wesentlich für den Erfindungsgedanken ist hierbei die relative Dehnung der Vorrichtung beziehungsweise von Bereichen der Vorrichtung im maximal belasteten Zustand beziehungsweise im maximal und im nominal belasteten Zustand.

Die selektive Beschichtung umfasst, wie beschrieben, jene Bereiche oder Abschnitte nicht, die eine Dehnung aufweisen, die mindestens den definierten Grenzwert erreicht. Im Idealfall bedeutet dies, dass die Beschichtung alle Bereiche umfasst, die unterhalb dieses Grenzwertes liegen.

In der Praxis hat sich gezeigt, dass es für die angestrebte Verbesserung der mechanischen Eigenschaften der Vorrichtung ausreichend ist, wenn der Abschnitt der Vorrichtung nicht beschichtet wird, der den Bereich mit der Dehnung umfasst, die mindestens den definierten Grenzwert erreicht. Bei einem Stent wird beispielsweise der entsprechende Abschnitt der jeweiligen Strebe nicht beschichtet.

Dabei ist bei der Bestimmung eines Abschnitts zu beachten, dass dieser nicht willkürlich groß festlegbar ist und somit unangemessen viele Bereiche umfasst, die unterhalb der definierten Grenzwerte für die Dehnung liegen. Entsprechend soll als Abschnitt ein Teil der Vorrichtung definiert sein, der genau diese Bereiche umfasst, die mindestens den definierten Grenzwert für die Dehnung erreichen. Ein Bereich, der mindestens den definierten Grenzwert erreicht, soll auch Dehnungsbereich genannt werden.

Am Beispiel einer stentartigen Vorrichtung wie beispielsweise einem Stent, einer Thrombektomievorrichtung oder einem Flow Diverter bedeutet dies, dass maximal der Abschnitt einer Strebe als Abschnitt definiert und entsprechend nicht beschichtet werden soll, der den Dehnungsbereich genau einschließt, also am Beginn des Dehnungsbereichs anfängt und am Ende des Dehnungsbereichs endet, wobei sich die Begriffe Beginn und Ende auf den Verlauf des Dehnungsbereichs entlang der Vorrichtung, also beispielsweise entlang einer Strebe, beziehen.

Sofern mehrere Dehnungsbereiche einem Abschnitt zuzuordnen sind, weil diese Dehnungsbereiche beispielsweise auf unterschiedlichen Seiten einer Strebe aber unmittelbar in diesem Abschnitt liegen, soll maximal der Teil der Strebe als Abschnitt definiert und nicht beschichtet werden, der alle diese Dehnungsbereiche genau einschließt, also am Beginn des ersten Dehnungsbereichs anfängt und am Ende des letzten Dehnungsbereichs endet, wobei Beginn und Ende auch Teil desselben Dehnungsbereichs sein können, wenn dieser sich nämlich über alle sonstigen Dehnungsbereichs des Abschnitts erstreckt.

Eine analoge Definition bietet sich für andere endovaskuläre Vorrichtungen an.

Dabei ist es für die Erfindung unerheblich, auf welche Art die endovaskuläre Vorrichtung aus Formgedächtnislegierungen hergestellt wurde. Die Vorrichtung kann beispielsweise durch Schneiden, insbesondere Laserschneiden, oder durch Flechten beispielsweise von Drähten hergestellt sein, wobei stärkere Dehnungen eher bei geschnittenen, beispielsweise aus einem Rohr lasergeschnittenen, Vorrichtungen zu erwarten sind. Grundsätzlich denkbar sind jedoch auch andere Arten der Herstellung der Vorrichtung, beispielsweise additive Verfahren, bei denen eine Struktur durch Hinzufügen von Material nach und nach aufgebaut wird. Ein derartiges additives Verfahren ist insbesondere der 3-D-Druck.

Der radial maximal belastete Zustand ist definitionsgemäß der Zustand der Vorrichtung, in dem die Vorrichtung ihre maximale radiale Kompression erfährt. Den radial maximal belasteten Zustand erfährt die Vorrichtung beispielsweise im Zuführkatheter vor und während einer Intervention. Ist die Vorrichtung beispielsweise für die Verwendung mittels eines Zuführkatheters mit einem Innendurchmesser von 0,53 mm vorgesehen, so erfährt die Vorrichtung bei der radialen Kompression auf einen Durchmesser von 0,53 mm ihren radial maximal belasteten Zustand. Die radiale Kompression auf einen Durchmesser von 0,53 mm ist für diese Vorrichtung der radial maximal belastete Zustand.

Sofern die Vorrichtung für die Verwendung mittels Zuführkathetern unterschiedlicher Innendurchmesser vorgesehen ist, so soll der radial maximal belastete Zustand für diese Vorrichtung der Zustand sein, den die Vorrichtung in dem Zuführkatheter mit dem kleinsten für diese Vorrichtung vorgesehenen Innendurchmesser einnimmt.

Sofern es angezeigt ist, den radial maximal belasteten Zustand der Vorrichtung nicht in Abhängigkeit von einem Zuführkatheter zu bestimmen, so kann der radial maximal belastete Zustand alternativ als der Zustand definiert sein, in dem die Vorrichtung die Grenze ihrer Pseudoelastizität erreicht und bei radialer Belastung gerade noch keine Bereiche aufweist, die dauerhaft, also nicht mehr reversibel, verformt sind. Eine solche Grenze ist bei einer Vorrichtung aus Formgedächtnislegierungen in der Regel dann erreicht, wenn diese bei radialer Kompression Bereiche aufweist, die eine Dehnung von 10 % gegenüber dem radial unbelasteten Zustand aufweisen.

Eine solche alternative Definition des radial maximal belasteten Zustands kann in bestimmten Fällen geeignet sein, eine weitere objektive Bezugsgröße zu schaffen.

Der radial nominal belastete Zustand ist definitionsgemäß der Zustand der Vorrichtung, in dem die Vorrichtung ihre nominale radiale Kompression erfährt. Diesen Zustand erfährt die Vorrichtung beispielsweise während der vorschriftsmäßigen Anwendung, beispielsweise bei der Implantation oder der sonstigen Verwendung in einem Gefäß, beispielsweise als Gefäßstütze, Filter oder zur Thrombektomie. Ist die Vorrichtung entsprechend für die Anwendung in Gefäßen mit einem Durchmesser von 2 mm vorgesehen, so erfährt die Vorrichtung bei einer radialen Kompression auf einen Durchmesser von 2 mm ihren radial nominal belasteten Zustand. Die radiale Kompression auf einen Durchmesser von 2 mm ist für diese Vorrichtung der radial nominal belastete Zustand.

Als radial belasteter Zustand einer Vorrichtung, sei es der radial nominal belastete Zustand oder der radial maximal belastete Zustand, soll immer ein Zustand mit einer gleichverteilten radialen Belastung der Vorrichtung durch äußere Krafteinwirkung verstanden werden, wie er beispielsweise in einem Blutgefäß oder einem Zuführkatheter oder modellhaft bei der gleichmäßigen Kompression der Vorrichtung innerhalb einer gleichförmigen geraden Röhre erreicht wird.

Der radial unbelastete Zustand ist definitionsgemäß der Zustand der Vorrichtung, in dem die Vorrichtung keine radiale Kompression erfährt. Diesen Zustand erfährt die Vorrichtung beispielsweise im frei expandierten Zustand außerhalb eines Gefäßes oder eines Katheters ohne äußere Restriktionen. Zu einer selektiven Beschichtung im Sinne der Erfindung führen alle solchen Beschichtungsverfahren, die geeignet sind, ein anwendungsfertiges Endprodukt mit einer nur teilweisen Beschichtung bereitzustellen. Dabei ist es unerheblich, wie die selektive Beschichtung des Endprodukts erreicht wird.

So kann beispielsweise im Beschichtungsprozess zunächst die gesamte Vorrichtung beschichtet und die Beschichtung anschließend teilweise mechanisch, chemisch oder elektrochemisch wieder entfernt werden. Auch können im Beschichtungsprozess Bereiche, die nicht beschichtet werden sollen, vorab mit verschiedenen Materialien wie Kunststoffen, Klebern oder Abdecklacken maskiert beziehungsweise abgedeckt werden. Entsprechende Maskierungen können nach der Beschichtung beispielsweise wieder entfernt werden oder auch an der Vorrichtung verbleiben. Außerdem können Hilfselemente innerhalb und/oder außerhalb der Vorrichtung angebracht werden, welche beim Beschichtungsvorgang als Schablone dienen.

Nicht zuletzt können gezielt nur die zu beschichtenden Teile beschichtet werden. Dem Fachmann sind verschiedene Möglichkeiten bekannt, die zu der gewünschten selektiven Beschichtung führen.

Die pseudoelastischen Legierungen beziehungsweise die Formgedächtnislegierungen können insbesondere Nitinol (NiTi) oder Nickel-Titan-Kupfer (NiTiCu) sein. Weitere pseudoelastische Legierungen sind dem Fachmann bekannt.

Das Material der röntgendichten Beschichtung kann ausgewählt sein aus der Gruppe umfassend Platin, Palladium, Platin-Iridium, Tantal, Gold und Wolfram. Die röntgendichte Beschichtung kann auch eine Kombination von zwei oder mehreren dieser Materialien oder Legierungen daraus umfassen. Entsprechend umfasst die röntgendichte Beschichtung mindestens eines der genannten Materialien. Weitere röntgendichte Materialien wählt der Fachmann je nach spezieller Anforderung aus und kombiniert diese gegebenenfalls mit den genannten Materialien.

Die Erfindung umfasst entsprechend ein Verfahren zur selektiven Beschichtung einer endovaskulären Vorrichtung aus pseudoelastischen Legierungen mit folgenden Schritten:
(A) Bereitstellen einer endovaskulären Vorrichtung aus pseudoelastischen Legierungen im radial unbelasteten Zustand;
(D) Komprimieren der endovaskulären Vorrichtung auf den radial maximal belasteten Zustand durch radiale Belastung der Vorrichtung;
(E) Bestimmen der Bereiche der endovaskulären Vorrichtung, die im radial maximal belasteten Zustand eine Dehnung von mindestens 5 % gegenüber dem radial unbelasteten expandierten Zustand aufweisen;
(F) Beschichten der endovaskulären Vorrichtung mit Ausnahme der in Schritt (E) bestimmten Bereiche.

In einer bevorzugten Ausführungsform umfasst das Verfahren zur selektiven Beschichtung einer endovaskulären Vorrichtung aus pseudoelastischen Legierungen weiterhin folgende Schritte:
(B) Komprimieren des endovaskulären Vorrichtung auf den radial nominal belasteten Zustand durch radiale Belastung der Vorrichtung;
(C) Bestimmen der Bereiche der endovaskulären Vorrichtung, die im radial nominal belasteten Zustand eine Dehnung von mindestens 5 % gegenüber der Vorrichtung im radial unbelasteten expandierten Zustand aufweisen;

Die Schritte (B) und (C) werden der Benennung entsprechend in der Regel nach Schritt (A) und vor Schritt (D) ausgeführt.

Die Beschichtung gemäß Schritt (F) wird bei dieser Variante des Verfahrens entsprechend unter Ausnehmung der in den Schritten (C) und (E) ermittelten Bereiche durchgeführt.

Die Schritte (B) und (C) können auch anstelle der Schritte (D) und (E) durchgeführt werden. Die Beschichtung gemäß Schritt (F) wird dann entsprechend unter Ausnehmung der in Schritt (C) ermittelten Bereiche durchgeführt.

Ein alternatives Verfahren zur selektiven Beschichtung einer endovaskulären Vorrichtung aus pseudoelastischen Legierungen umfasst folgende Schritte:
(A') Bereitstellen einer endovaskulären Vorrichtung aus pseudoelastischen Legierungen im radial unbelasteten Zustand;
(D') Komprimieren der endovaskulären Vorrichtung auf den radial maximal belasteten Zustand durch radiale Belastung der Vorrichtung;
(E') Bestimmen der Bereiche der endovaskulären Vorrichtung, die im radial maximal belasteten Zustand eine Dehnung von mindestens 5 % gegenüber dem radial unbelasteten expandierten Zustand aufweisen, und anschließendes Bestimmen der Abschnitte der endovaskulären Vorrichtung, die die entsprechenden Bereiche definierter Dehnung umfassen;
(F') Beschichten der endovaskulären Vorrichtung mit Ausnahme der in Schritt (E') bestimmten Abschnitte.

In einer bevorzugten Ausführungsform umfasst das Verfahren zur selektiven Beschichtung einer endovaskulären Vorrichtung aus pseudoelastischen Legierungen weiterhin folgende Schritte:
(B') Komprimieren der endovaskulären Vorrichtung auf den radial nominal belasteten Zustand durch radiale Belastung der Vorrichtung;
(C') Bestimmen der Bereiche der endovaskulären Vorrichtung, die im radial nominal belasteten Zustand eine Dehnung von mindestens 5 % gegenüber der Vorrichtung im radial unbelasteten expandierten Zustand aufweisen und anschließendes Bestimmen der Abschnitte der endovaskulären Vorrichtung, die die entsprechenden Bereiche definierter Dehnung umfassen;

Die Schritte (B') und (C') werden der Benennung entsprechend in der Regel nach Schritt (A') und vor Schritt (D') ausgeführt.

Die Beschichtung gemäß Schritt (F') wird bei dieser Variante des Verfahrens unter Ausnehmung der in den Schritten (C') und (E') ermittelten Abschnitte durchgeführt.

Die Schritte (B') und (C') können auch anstelle der Schritte (D') und (E') durchgeführt werden. Die Beschichtung gemäß Schritt (F') wird dann unter Ausnehmung der in Schritt (C') ermittelten Abschnitte durchgeführt.

Es ist dem Fachmann klar, dass die Schritte (B) und (C) und die Schritte (D) und (E) auch in der Reihenfolge gegeneinander ausgetauscht werden können, also nach Schritt (A) zunächst die Schritte (D) und (E) und anschließend die Schritte (B) und (C) ausgeführt werden. Gleiches gilt für die Schritte (B') und (C') und die Schritte (D') und (E'). Auch diese können in der Reihenfolge gegeneinander ausgetauscht werden, sodass nach Schritt (A') zunächst die Schritte (D') und (E') und anschließend die Schritte (B') und (C') ausgeführt werden.

Die Dehnung gemäß der Schritte (C) und (E) beziehungsweise gemäß der Schritte (C') und (E') kann vorzugsweise mindestens 3 %, noch bevorzugter mindestens 1,5 % und insbesondere mindestens 1 % gegenüber dem radial unbelasteten Zustand betragen.

Die Vorrichtung kann zur Komprimierung auf den radial nominal belasteten Zustand gemäß Verfahrensschritt (B) oder (B') vorzugsweise in eine gerade Röhre mit einem entsprechenden Innendurchmesser eingebracht werden. Der Innendurchmesser der Röhre soll dem Außendurchmesser der Vorrichtung im radial nominal belasteten Zustand entsprechen. Die Röhre ist vorteilhafterweise starr und gibt bei der Expansion der Vorrichtung nicht nach. Hierdurch können vergleichbare Ergebnisse gewonnen werden.

Die Vorrichtung kann zur Komprimierung auf den radial maximal belasteten Zustand gemäß Verfahrensschritt (D) oder (D') in eine gerade Röhre mit einem entsprechenden Innendurchmesser eingebracht werden. Der Innendurchmesser der Röhre soll dem Außendurchmesser der Vorrichtung im radial maximal belasteten Zustand entsprechen. Die Röhre ist vorteilhafterweise starr und gibt bei der Expansion der Vorrichtung nicht nach. Hierdurch können vergleichbare Ergebnisse gewonnen werden.

Grundsätzlich ist es auch denkbar, dass die Verfahrensschritte (A) bis (E) beziehungsweise (A') bis (E') in einer Computersimulation durchgeführt werden, beispielsweise mittels der Finite-Elemente-Methode (FEM), die den beschriebenen realen Versuchsaufbau modelliert.

Die Erfindung umfasst entsprechend auch eine endovaskuläre Vorrichtung mit einer Beschichtung erhältlich durch das erfinderische Verfahren.

Die Erfindung umfasst weiterhin eine Kombination umfassend eine erfindungsgemäß beschichtete endovaskuläre Vorrichtung und einen Zuführkatheter für diese Vorrichtung.

Eine erfindungsgemäße Vorrichtung mit der erfindungsgemäßen selektiven Beschichtung weist gegenüber dem Stand der Technik den Vorteil auf, dass die Vorrichtung durch eine nahezu vollständige Beschichtung auch nahezu vollständig im Röntgenbild sichtbar ist, ohne dass durch die Beschichtung jedoch wichtige mechanische Eigenschaften wie RRF und COF in einem relevanten Maß beeinträchtigt werden.

Die Effektivität der vorgeschlagenen selektiven Beschichtung wird nachfolgend anhand eines Beispiels verdeutlicht.

### Beispiel

Bei endovaskulären Vorrichtungen, wie beispielsweise Stent-Systemen, aus Formgedächtnislegierungen nimmt die COF mit zunehmender Expansion bei einem entsprechend stetig größer werdenden Stent-Durchmesser kontinuierlich ab. Bei solchen Vorrichtungen aus Formgedächtnislegierungen, die vollständig mit röntgensichtbaren Materialien wie beispielsweise Gold beschichtet sind, verschlechtert sich die COF erheblich, sie nimmt also ab. Durch ein selektives Beschichten der Bereiche, die nicht wesentlich zur COF beitragen, kann dieser Effekt minimiert werden.

Bei den vorliegenden Beispielen wurde eine selektive Beschichtung auf die Bereiche beziehungsweise Abschnitte der Vorrichtung aus einer Formgedächtnislegierung aufgetragen, die gemäß Definition eine Dehnung von unter 1,5 % im radial maximal belasteten beziehungsweise im radial nominal belasteten Zustand zeigten.

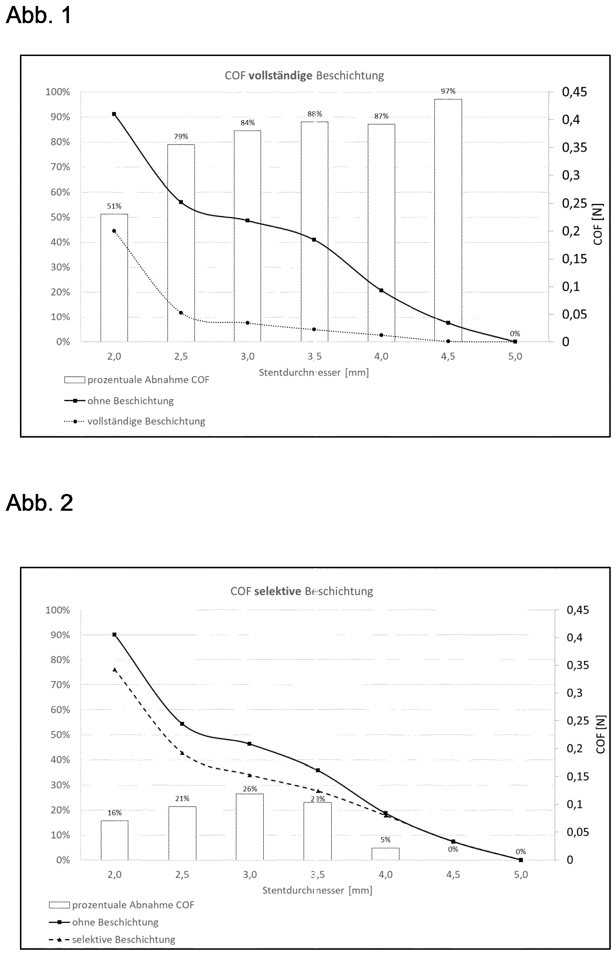

Abbildung 1 zeigt im Vergleich den Verlauf der COF bei einer vollständig beschichteten endovaskulären Vorrichtung aus einer Formgedächtnislegierung (untere Kurve) und einer vollständig unbeschichteten endovaskulären Vorrichtung aus einer Formgedächtnislegierung (obere Kurve), die von ihrem radial nominal belasteten Zustand, bei dem der Durchmesser der Vorrichtung 2 mm beträgt, auf ihren radial unbelasteten Zustand, bei dem der Durchmesser der Vorrichtung 5 mm beträgt, expandiert wird. Dabei ist gut zu erkennen, dass die COF der vollständig beschichteten Vorrichtung mit einem Verlust zwischen 51 % und 97 % deutlich unter der der unbeschichteten Vorrichtung liegt.

Abbildung 2 zeigt im Vergleich den Verlauf der COF bei einer erfindungsgemäß selektiv beschichteten Vorrichtung aus einer Formgedächtnislegierung (untere Kurve) und einer vollständig unbeschichteten Vorrichtung aus einer Formgedächtnislegierung (obere Kurve) die von ihrem radial nominal belasteten Zustand, bei dem der Durchmesser der Vorrichtung 2 mm beträgt, auf ihren radial unbelasteten Zustand, bei dem der Durchmesser der Vorrichtung 5 mm beträgt, expandiert wird. Dabei ist gut zu erkennen, dass die COF der selektiv beschichteten Vorrichtung mit einem maximalen Verlust der COF von nur 26 % deutlich geringfügiger unter der der unbeschichteten Vorrichtung liegt.

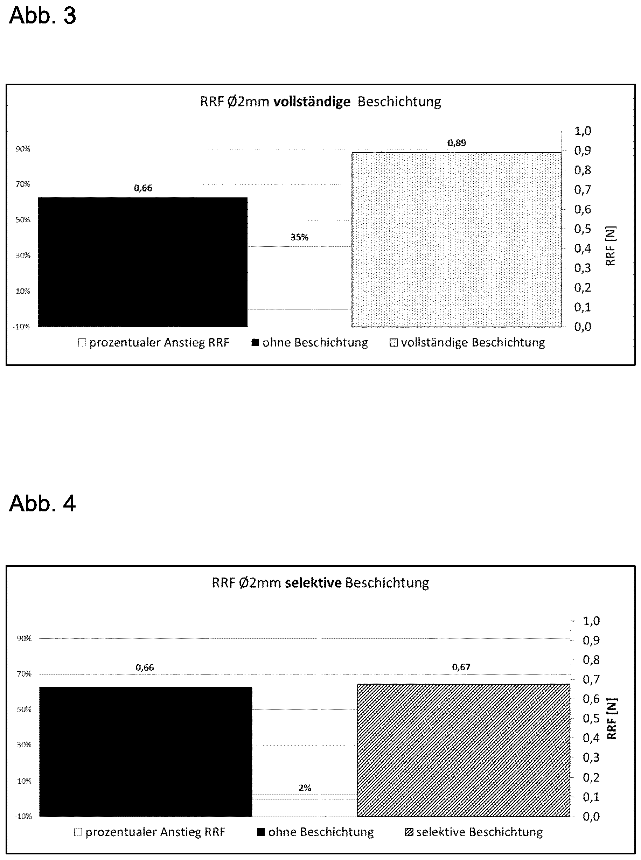

Abbildung 3 zeigt im Vergleich die Zunahme der RRF bei einer vollständig beschichteten endovaskulären Vorrichtung aus einer Formgedächtnislegierung (rechter Balken) und einer unbeschichteten endovaskulären Vorrichtung aus einer Formgedächtnislegierung (linker Balken), jeweils im radial nominal belasteten Zustand, der bei einem Durchmesser von 2 mm liegt. Dabei ist gut zu erkennen, dass die RRF der vollständig beschichteten Vorrichtung mit einem prozentualen Anstieg von 35 % deutlich über der der unbeschichteten Vorrichtung liegt.

Abbildung 4 zeigt im Vergleich die Zunahme der RRF bei einer selektiv beschichteten endovaskulären Vorrichtung aus einer Formgedächtnislegierung (rechter Balken) und einer unbeschichteten endovaskulären Vorrichtung aus einer Formgedächtnislegierung (linker Balken), jeweils im radial nominal belasteten Zustand, der bei einem Durchmesser von 2 mm liegt. Dabei ist gut zu erkennen, dass die RRF der erfindungsgemäß selektiv beschichteten Vorrichtung mit einem prozentualen Anstieg von nur 2 % nur unwesentlich über der der unbeschichteten Vorrichtung liegt.

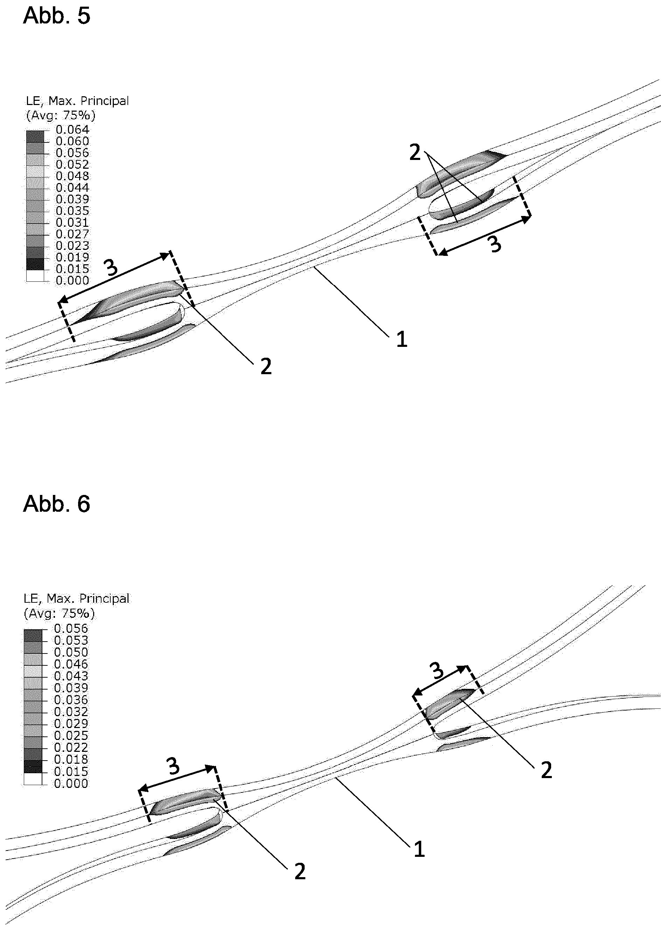

Abbildung 5 zeigt die erfindungsgemäße Bestimmung der durch die Kompression belasteten Bereiche einer endovaskulären Vorrichtung aus einer Formgedächtnislegierung. Vorliegend wurden die Daten zur Dehnung der Bereiche 2 der Vorrichtung beziehungsweise der Streben 1 in einer Computersimulation mittels der Finite-Elemente-Methode (FEM) ermittelt. Die markierten Bereiche 2 stellen die Bereiche der Vorrichtung beziehungsweise der Streben 1 dar, deren Dehnung bei einer Kompression im Mikrokatheter, also bei dem radial maximal belasteten Zustand, mindestens 1,5 % gegenüber dem radial unbelasteten Zustand beträgt. Diese Bereiche 2 werden bei einer erfindungsgemäßen Beschichtung ausgespart.

Weiterhin zeigt Abbildung 5 die aus den Bereichen 2 abgeleiteten Abschnitte 3 der Streben 1, die alternativ bei der selektiven Beschichtung ausgespart werden können.

Abbildung 6 zeigt die erfindungsgemäße Bestimmung der durch die Kompression belasteten Bereiche 2 beziehungsweise Streben 1 einer endovaskulären Vorrichtung aus einer Formgedächtnislegierung. Vorliegend wurden die Daten zur Dehnung der Bereiche 2 beziehungsweise der Streben 1 der endovaskulären Vorrichtung in einer Computersimulation mittels der Finite-Elemente-Methode (FEM) ermittelt. Die markierten Bereiche 2 stellen die Bereiche 2 beziehungsweise der Streben 1 der endovaskulären Vorrichtung dar, deren Dehnung bei einer Kompression im Gefäß, also im radial nominal belasteten Zustand, mindestens 1,5 % gegenüber dem radial unbelasteten Zustand beträgt. Diese Bereiche 2 werden bei einer erfindungsgemäßen Beschichtung ausgespart.

Weiterhin zeigt Abbildung 6 die aus den Bereichen 2 abgeleiteten Abschnitte 3 der Streben 1, die alternativ bei der selektiven Beschichtung ausgespart werden können.

Die Abbildungen 1 bis 6 beziehen sich jeweils auf lasergeschnittene endovaskuläre Vorrichtungen.

## Patentansprüche

1. Endovaskuläre Vorrichtung aus pseudoelastischen Legierungen umfassend eine selektive Beschichtung aus röntgendichten Materialien, **dadurch gekennzeichnet, dass** die Beschichtung nur Bereiche oder Abschnitte der endovaskulären Vorrichtung umfasst, die im radial maximal belasteten Zustand der Vorrichtung eine Dehnung von unter 5 % gegenüber dem radial unbelasteten Zustand der Vorrichtung aufweisen.

2. Endovaskuläre Vorrichtung aus pseudoelastischen Legierungen umfassend eine selektive Beschichtung aus röntgendichten Materialien gemäß Anspruch 1, **dadurch gekennzeichnet, dass** die Beschichtung nur Bereiche oder Abschnitte der endovaskulären Vorrichtung umfasst, die sowohl im radial maximal belasteten Zustand als auch im radial nominal belasteten Zustand der Vorrichtung eine Dehnung von unter 5 % gegenüber dem radial unbelasteten Zustand der Vorrichtung aufweisen.

3. Endovaskuläre Vorrichtung nach Anspruch 1 oder 2, **dadurch gekennzeichnet, dass** die Dehnung im radial maximal beziehungsweise im radial nominal belasteten Zustand unter 3 %, vorzugsweise unter 1,5 % und insbesondere unter 1 % gegenüber dem radial unbelasteten Zustand liegt.

4. Endovaskuläre Vorrichtung nach einem der vorherigen Ansprüche, **dadurch gekennzeichnet, dass** die Beschichtung mindestens ein röntgendichtes Material ausgewählt aus der Gruppe Platin, Palladium, Platin-Iridium, Tantal, Gold und Wolfram umfasst.

5. Endovaskuläre Vorrichtung nach einem der vorherigen Ansprüche, **dadurch gekennzeichnet, dass** das pseudoelastische Material NiTi oder NiTiCu umfasst.

6. Verfahren zur selektiven Beschichtung einer endovaskulären Vorrichtung aus pseudoelastischen Legierungen umfassend folgende Schritte:
(A) Bereitstellen einer endovaskulären Vorrichtung aus pseudoelastischen Legierungen im radial unbelasteten Zustand;
(D) Komprimieren der endovaskulären Vorrichtung auf den radial maximal belasteten Zustand durch radiale Belastung der Vorrichtung;
(E) Bestimmen der Bereiche der endovaskulären Vorrichtung, die im radial maximal belasteten Zustand eine Dehnung von mindestens 5 % gegenüber dem radial unbelasteten expandierten Zustand aufweisen;
(F) Beschichten der endovaskulären Vorrichtung mit Ausnahme der in Schritt (E) bestimmten Bereiche.

7. Verfahren zur selektiven Beschichtung einer endovaskulären Vorrichtung aus pseudoelastischen Legierungen gemäß Anspruch 6, weiterhin umfassend folgende Schritte:
(B) Komprimieren der endovaskulären Vorrichtung auf den radial nominal belasteten Zustand durch radiale Belastung der Vorrichtung;
(C) Bestimmen der Bereiche der endovaskulären Vorrichtung, die im radial nominal belasteten Zustand eine Dehnung von mindestens 5 % gegenüber der Vorrichtung im radial unbelasteten expandierten Zustand aufweisen;
wobei die Beschichtung gemäß Schritt (F) unter Ausnehmung der in den Schritten (C) und (E) bestimmten Bereiche durchgeführt wird.

8. Verfahren zur selektiven Beschichtung einer endovaskulären Vorrichtung aus pseudoelastischen Legierungen gemäß Anspruch 7, wobei die Schritte (D) und (E) nach Schritt (A) und vor den Schritten (B) und (C) durchgeführt werden.

9. Verfahren zur selektiven Beschichtung einer endovaskulären Vorrichtung aus pseudoelastischen Legierungen umfassend folgende Schritte:
(A') Bereitstellen einer endovaskulären Vorrichtung aus pseudoelastischen Legierungen im radial unbelasteten Zustand;
(D') Komprimieren der endovaskulären Vorrichtung auf den radial maximal belasteten Zustand durch radiale Belastung der Vorrichtung;
(E') Bestimmen der Bereiche der endovaskulären Vorrichtung, die im radial maximal belasteten Zustand eine Dehnung von mindestens 5 % gegenüber dem radial unbelasteten expandierten Zustand aufweisen, und anschließendes Bestimmen der Abschnitte der endovaskulären Vorrichtung, die die entsprechenden Bereiche definierter Dehnung umfassen;
(F') Beschichten der endovaskulären Vorrichtung mit Ausnahme der in Schritt (E') bestimmten Abschnitte.

10. Verfahren zur selektiven Beschichtung einer endovaskulären Vorrichtung aus pseudoelastischen Legierungen gemäß Anspruch 9, weiterhin umfassend folgende Schritte:
(B') Komprimieren der endovaskulären Vorrichtung auf den radial nominal belasteten Zustand durch radiale Belastung der Vorrichtung;
(C') Bestimmen der Bereiche der endovaskulären Vorrichtung, die im radial nominal belasteten Zustand eine definierte Dehnung von mindestens 5 % gegenüber der Vorrichtung im radial unbelasteten expandierten Zustand aufweisen, und anschließendes Bestimmen der Abschnitte der endovaskulären Vorrichtung, die die entsprechenden Bereiche definierter Dehnung umfassen;
wobei die Beschichtung gemäß Schritt (F') unter Ausnehmung der in den Schritten (C') und (E') ermittelten Abschnitte durchgeführt wird.

11. Verfahren zur selektiven Beschichtung einer endovaskulären Vorrichtung aus pseudoelastischen Legierungen gemäß Anspruch 10, wobei die Schritte (D') und (E') nach Schritt (A') und vor den Schritten (B') und (C') durchgeführt werden.

12. Verfahren zur selektiven Beschichtung einer endovaskulären Vorrichtung aus pseudoelastischen Legierungen gemäß einem der Ansprüche 6 bis 11, **dadurch gekennzeichnet, dass** die Vorrichtung zur Komprimierung auf den radial nominal belasteten Zustand gemäß Verfahrensschritt (B) oder (B') in eine gerade Röhre mit einem entsprechenden Innendurchmesser eingebracht wird.

13. Verfahren zur selektiven Beschichtung einer endovaskulären Vorrichtung aus pseudoelastischen Legierungen gemäß einem der Ansprüche 6 bis 11, **dadurch gekennzeichnet, dass** die Vorrichtung zur Komprimierung auf den radial maximal belasteten Zustand gemäß Verfahrensschritt (D) oder (D') in eine gerade Röhre mit einem entsprechenden Innendurchmesser eingebracht wird.

14. Verfahren zur selektiven Beschichtung einer endovaskulären Vorrichtung aus pseudoelastischen Legierungen gemäß einem der Ansprüche 6 bis 11, **dadurch gekennzeichnet, dass** die Dehnung gemäß den Schritten (C) und (E) beziehungsweise (C') und (E') mindestens 3 %, vorzugsweise mindestens 1,5 % und insbesondere mindestens 1 % gegenüber dem radial unbelasteten Zustand beträgt.

15. Verfahren zur selektiven Beschichtung einer endovaskulären Vorrichtung aus pseudoelastischen Legierungen gemäß einem der Ansprüche 6 bis 14, **dadurch gekennzeichnet, dass** die Verfahrensschritte (A) bis (E) beziehungsweise (A') bis (E') in einer Computersimulation durchgeführt werden, beispielsweise mittels der Finite-Elemente-Methode (FEM).

16. Endovaskuläre Vorrichtung mit einer Beschichtung erhältlich durch ein Verfahren gemäß einem der Ansprüche 6 bis 15.

17. Kombination umfassend eine endovaskuläre Vorrichtung gemäß einem der Ansprüche 1 bis 5 oder 16 und einen Zuführkatheter.

## Claims

1. Endovascular device made of pseudoelastic alloys and comprising a selective coating of radiopaque materials, **characterised in that** the coating covers only regions or portions of the endovascular device, which regions or portions, when in the radially maximum loaded state, have an elongation of less than 5% compared to the radially non-loaded state of the device.

2. Endovascular device made of pseudoelastic alloys and comprising a selective coating of radiopaque materials according to claim 1, **characterised in that** the coating covers only regions or portions of the endovascular device, which regions or portions, when in both the radially maximum loaded state and in the radially nominally loaded state, have an elongation of less than 5% compared to the radially non-loaded state of the device.

3. Endovascular device according to claim 1 or 2, **characterised in that** the elongation in the radially maximum or radially nominally loaded state is less than 3%, preferably less than 1.5% and in particular less than 1% compared to the radially non-loaded state.

4. Endovascular device according to any one of the preceding claims, **characterised in that** the coating comprises at least one radiopaque material selected from the group of platinum, palladium, platinum iridium, tantalum, gold and tungsten.

5. Endovascular device according to any one of the preceding claims, **characterised in that** the pseudoelastic material comprises NiTi or NiTiCu.

6. Method for selectively coating an endovascular device made of pseudoelastic alloys comprising the following steps:
(A) providing an endovascular device made of pseudoelastic alloys in the radially non-loaded state;
(D) compressing the endovascular device to the radially maximum loaded state by radial loading of the device;
(E) determining the regions of the endovascular device that have an elongation of at least 5% in the radially maximum loaded state compared to the radially non-loaded expanded state;
(F) coating the endovascular device except for the regions determined in step (E).

7. Method for selectively coating an endovascular device made of pseudoelastic alloys according to claim 6, further comprising the following steps:
(B) compressing the endovascular device to the radially nominally loaded state by radial loading of the device;
(C) determining the regions of the endovascular device that have an elongation of at least 5% in the radially nominally loaded state compared to the device in the radially non-loaded expanded state;
wherein the coating according to step (F) is carried out excluding the regions determined in steps (C) and (E).

8. Method for selectively coating an endovascular device made of pseudoelastic alloys according to claim 7, wherein steps (D) and (E) are carried out after step (A) and before steps (B) and (C).

9. Method for selectively coating an endovascular device made of pseudoelastic alloys comprising the following steps:
(A') providing an endovascular device made of pseudoelastic alloys in the radially non-loaded state;
(D') compressing the endovascular device to the radially maximum loaded state by radial loading the device;
(E') determining the regions of the endovascular device that have an elongation of at least 5% in the radially maximum loaded state compared to the radially non-loaded expanded state, and subsequently determining the sections of the endovascular device comprising the corresponding regions of defined elongation;
(F') coating the endovascular device except for the sections determined in step (E').

10. Method for selectively coating an endovascular device made of pseudoelastic alloys according to claim 9, further comprising the following steps:
(B') compressing the endovascular device to the radially nominally loaded state by radial loading of the device;
(C') determining the regions of the endovascular device that have a defined elongation of at least 5% in the radially nominally loaded state compared to the device in the radially non-loaded expanded state, and subsequently determining the sections of the endovascular device comprising the corresponding regions of defined elongation;
wherein the coating according to step (F') is carried out excluding the sections determined in steps (C') and (E').

11. Method for selectively coating an endovascular device made of pseudoelastic alloys according to claim 10, wherein steps (D') and (E') are carried out after step (A') and before steps (B') and (C').

12. Method for selectively coating an endovascular device made of pseudoelastic alloys according to any one of claims 6 to 11, **characterised in that** the device is inserted into a straight tube with a corresponding inner diameter for compression to the radially nominally loaded state according to method step (B) or (B').

13. Method for selectively coating an endovascular device made of pseudoelastic alloys according to any one of claims 6 to 11, **characterised in that** the device is inserted into a straight tube with a corresponding inner diameter for compression to the radially maximum loaded state according to method step (D) or (D').

14. Method for selectively coating an endovascular device made of pseudoelastic alloys according to any one of claims 6 to 11, **characterised in that** the elongation according to steps (C) and (E) or (C') and (E') is at least 3%, preferably at least 1.5% and in particular at least 1% compared to the radially non-loaded state.

15. Method for selectively coating an endovascular device made of pseudoelastic alloys according to any one of claims 6 to 14, **characterised in that** the method steps (A) to (E) or (A') to (E') are carried out in a computer simulation, for example using the finite element method (FEM).

16. Endovascular device having a coating that can be obtained by a method according to any one of claims 6 to 15.

17. Combination comprising an endovascular device according to any one of claims 1 to 5 or 16 and a feed catheter.

## Revendications

1. Dispositif endovasculaire en alliages pseudo-élastiques comprenant un revêtement sélectif en matériaux radio-opaques, **caractérisé en ce que** le revêtement ne comprend que des zones ou des sections du dispositif endovasculaire qui, à l'état de contrainte radiale maximale du dispositif, présentent un allongement inférieur à 5 % par rapport à l'état sans contrainte radiale du dispositif.

2. Dispositif endovasculaire en alliages pseudo-élastiques comprenant un revêtement sélectif en matériaux radio-opaques selon la revendication 1, **caractérisé en ce que** le revêtement ne comprend que des zones ou des sections du dispositif endovasculaire qui, tant à l'état de contrainte radiale maximale qu'à l'état de contrainte radiale nominale du dispositif, présentent un allongement inférieur à 5 % par rapport à l'état sans contrainte radiale du dispositif.

3. Dispositif endovasculaire selon la revendication 1 ou 2, **caractérisé en ce que** l'allongement à l'état de contrainte radiale maximale ou à l'état de contrainte radiale nominale est inférieur à 3 %, de préférence inférieur à 1,5 % et notamment inférieur à 1 % par rapport à l'état sans contrainte radiale.

4. Dispositif endovasculaire selon l'une quelconque des revendications précédentes, **caractérisé en ce que** le revêtement comprend au moins un matériau radio-opaque choisi dans le groupe constitué par le platine, le palladium, le platine-iridium, le tantale, l'or et le tungstène.

5. Dispositif endovasculaire selon l'une quelconque des revendications précédentes, **caractérisé en ce que** le matériau pseudo-élastique comprend du NiTi ou du NiTiCu.

6. Procédé de revêtement sélectif d'un dispositif endovasculaire en alliages pseudo-élastiques, comprenant les étapes suivantes :
(A) la fourniture d'un dispositif endovasculaire en alliages pseudo-élastiques à l'état sans contrainte radiale ;
(D) la compression du dispositif endovasculaire à l'état de contrainte radiale maximale par contrainte radiale au dispositif ;
(E) la détermination des zones du dispositif endovasculaire qui, à l'état de contrainte radiale maximale, présentent un allongement d'au moins 5 % par rapport à l'état expansé sans contrainte radiale ;
(F) le revêtement du dispositif endovasculaire à l'exception des zones déterminées à l'étape (E).

7. Procédé de revêtement sélectif d'un dispositif endovasculaire en alliages pseudo-élastiques selon la revendication 6, comprenant en outre les étapes suivantes :
(B) la compression du dispositif endovasculaire à l'état de contrainte radiale nominale par contrainte radiale du dispositif ;
(C) la détermination des zones du dispositif endovasculaire qui, à l'état de contrainte radiale nominale, présentent un allongement d'au moins 5 % par rapport au dispositif à l'état expansé sans contrainte radiale ;
le revêtement selon l'étape (F) étant réalisé à l'exception des zones déterminées aux étapes (C) et (E).

8. Procédé de revêtement sélectif d'un dispositif endovasculaire en alliages pseudo-élastiques selon la revendication 7, les étapes (D) et (E) étant réalisées après l'étape (A) et avant les étapes (B) et (C).

9. Procédé de revêtement sélectif d'un dispositif endovasculaire en alliages pseudo-élastiques, comprenant les étapes suivantes :
(A') la fourniture d'un dispositif endovasculaire en alliages pseudo-élastiques à l'état sans contrainte radiale ;
(D') la compression du dispositif endovasculaire à l'état de contrainte radiale maximale par contrainte radiale du dispositif ;
(E') la détermination des zones du dispositif endovasculaire qui, à l'état de contrainte radiale maximale, présentent un allongement d'au moins 5 % par rapport à l'état expansé sans contrainte radiale, puis la détermination des sections du dispositif endovasculaire qui comprennent les zones correspondantes d'allongement défini ;
(F') le revêtement du dispositif endovasculaire à l'exception des sections déterminées à l'étape (E').

10. Procédé de revêtement sélectif d'un dispositif endovasculaire en alliages pseudo-élastiques selon la revendication 9, comprenant en outre les étapes suivantes :
(B') la compression du dispositif endovasculaire à l'état de contrainte radiale nominale par contrainte radiale du dispositif ;
(C') la détermination des zones du dispositif endovasculaire qui, à l'état de contrainte radiale nominale, présentent un allongement défini d'au moins 5 % par rapport au dispositif à l'état expansé sans contrainte radiale, puis la détermination des sections du dispositif endovasculaire qui comprennent les zones correspondantes d'allongement défini ;
le revêtement selon l'étape (F') étant réalisé à l'exception des sections déterminées dans les étapes (C') et (E').

11. Procédé de revêtement sélectif d'un dispositif endovasculaire en alliages pseudo-élastiques selon la revendication 10, les étapes (D') et (E') étant réalisées après l'étape (A') et avant les étapes (B') et (C').

12. Procédé de revêtement sélectif d'un dispositif endovasculaire en alliages pseudo-élastiques selon l'une quelconque des revendications 6 à 11, **caractérisé en ce que** le dispositif est placé dans un tube droit d'un diamètre intérieur correspondant pour la compression à l'état de contrainte radiale nominale selon l'étape de procédé (B) ou (B').

13. Procédé de revêtement sélectif d'un dispositif endovasculaire en alliages pseudo-élastiques selon l'une quelconque des revendications 6 à 11, **caractérisé en ce que** le dispositif est placé dans un tube droit d'un diamètre intérieur correspondant pour la compression à l'état de contrainte radiale maximale selon l'étape (D) ou (D') du procédé.

14. Procédé de revêtement sélectif d'un dispositif endovasculaire en alliages pseudo-élastiques selon l'une quelconque des revendications 6 à 11, **caractérisé en ce que** l'allongement selon les étapes (C) et (E) ou (C') et (E') est d'au moins 3 %, de préférence d'au moins 1,5 % et notamment d'au moins 1 % par rapport à l'état sans contrainte radiale.

15. Procédé de revêtement sélectif d'un dispositif endovasculaire en alliages pseudo-élastiques selon l'une quelconque des revendications 6 à 14, **caractérisé en ce que** les étapes de procédé (A) à (E) ou (A') à (E') sont réalisées dans une simulation informatique, par exemple au moyen de la méthode des éléments finis (FEM).

16. Dispositif endovasculaire avec un revêtement pouvant être obtenu par un procédé selon l'une quelconque des revendications 6 à 15.

17. Combinaison comprenant un dispositif endovasculaire selon l'une quelconque des revendications 1 à 5 ou 16 et un cathéter d'alimentation.
